# EUROPEAN PATENT APPLICATION

(11) **EP 4 036 223 A1**
(43) Date of publication of application: **03.08.2022**
(21) Application number: 22155051.0
(22) Date of filing: 10.05.2017
(51) Int. Cl.: C12N 5/078, C12N 15/11, C12N 15/63, C12N 15/86, A61P 31/18

(54) **LENTIVIRAL DELIVERY OF CAS CONSTRUCTS FOR THE TREATMENT AND PREVENTION OF HIV-1 INFECTIONS**

(30) Priority: 10.05.2016 US 201662334313 P
(62) Divisional of application: 17796801.3
(71) Applicant: United States Government as Represented by The Department of Veterans Affairs, Washington, DC 20420 (US); Dartmouth College, Hanover, NH 03755 (US)
(72) Inventor: HOWELL, Alexandra, Washington, DC 20420 (US); ESZTERHAS, Susan, Washington, DC 20420 (US); LUIKART, Bryan Weston, Hanover, NH 03755 (US)
(74) Representative: Barker Brettell LLP

(57) **Abstract**

Compositions, methods and systems that inactivate proviral HIV-1 by genome editing to excise viral sequences, thereby inactivating production of nascent virus. In some embodiments, proviral DNA is excised by targeting the LTR region of integrated viral DNA using a CRISPR/Cas9 lentiviral construct. Use of the lentiviral construct advantageously permits transduction of both dividing and non-dividing target cells.

## Description

### Field of the Invention

The present invention generally relates to the field of biotechnology. More specifically, the invention relates to genome editing technology. Still more specifically, the invention relates to lentiviral-based transduction of cells with a CRISPR/Cas9 system that inactivates HIV-1.

### Government Support

This invention was made with government support under NIH SBIR Grant 1R43AI110285-01, and Merit Review Award No. 1I01BX000260-01, awarded by the Veteran's Administration and R01 MH097949 (BWL) from the NIMH. The United States government has certain rights in the invention.

### Reference to Sequence Listing

A Sequence Listing submitted as in ASCII text file via EFS-Web is hereby incorporated by reference in accordance with 35 U.S.C. § 1.52(e). The name of the ASCII text file for the Sequence Listing is "2017-05-10 Sequence Listing As-Filed 118431-027WO1.TXT", the date of creation of the ASCII text file is May 10, 2017, and the size of the ASCII text file is 3 KB.

### Background

Current anti-retroviral therapy (ART) for HIV-1 infection effectively disrupts critical steps in the viral lifecycle to block production of new virions by infected cells. Despite their efficacy, however, the drugs used in ART do not target the integrated provirus which contains the structural and regulatory genes used for synthesizing new viral particles. When ART is withdrawn, viral resurgence from the latent HIV infected cell reservoir leads to increased plasma viral loads and new cycles of target cell infection. In addition, ART does not kill HIV-infected cells. This means that HIV-infected patients must remain on ART for life, since even short periods of treatment interruption have been shown to lead to increased mortality. Long-term use of these drugs requires careful monitoring due to the risk of organ toxicity and the potential for the induction of viral resistance.

A prokaryotic innate immune mechanism, termed CRISPR/Cas, was recently modified to allow specific targeting and modification of genes in the eukaryotic genome. In prokaryotes, a single-stranded RNA molecule together with an endonuclease termed Cas blocks pathogen invasion by cleaving the genomes of invading viruses (*see* Marraffini et al., Nat. Rev. Genetics 11:181-190 (2010)). In 2012, Charpentier and Doudna successfully modified the sequence for the type II bacterial Cas gene (Cas9) to function in eukaryotic cells. Shortly thereafter, genomic engineering using a guide RNA (gRNA) and Cas9 to cleave and disable eukaryotic genes was successfully reported by several groups in diverse cell systems. In eukaryotes, a guide RNA complementary to the genomic target DNA sequence cooperates with Cas9 to disrupt a specific gene by inducing a doublestranded cleavage cut that is imperfectly repaired. This leads to insertions and deletions (indels) at the site of cleavage, which in turn leads to aberrant transcription and translation.

The CRISPR/Cas technique has been applied to an HIV system by transfecting human cell lines with plasmids expressing the guide RNA and Cas9 genes that cleaved a single region in a model HIV proviral sequence carried on a plasmid (*see* Ebina et al., Sci. Rep. 3:2510 (2013)). Shortly thereafter, it was demonstrated that use of multiple guide RNAs targeted to both the 5' and 3' ends of the provirus completely excised the entire HIV proviral genome in cell lines, and protected the cells from a subsequent reinfection (*see* Hu et al., Proc. Natl. Acad. Sci. USA 111:11461-11466 (2014)).

Challenges remain, however, in the delivery of guide RNA and Cas9 genes, especially to resting leukocytes either *in vivo* or *ex vivo* as a therapeutic modality for treating HIV-infected patients. It is clear that delivery of plasmid DNA is not a tenable option. Therefore, what is needed is a system and method that overcomes these significant problems found in the conventional practices as described above.

### Summary

In one aspect, the invention relates to a lentiviral vector, such as "g-LV" and the like described herein, harboring nucleic acids encoding one or more guide RNA, wherein said guide RNA hybridizes with a target HIV-1 DNA sequence, and a Clustered Regularly Interspaced Short Palindromic Repeats-Associated (Cas) protein. The encoded Cas protein preferably is a Cas9 protein.

In another aspect, the invention relates to a kit containing a lentiviral vector that contains (a) one or more guide RNA, or nucleic acids encoding said one or more guide RNA, wherein said guide RNA hybridizes with a target HIV-1 DNA sequence; and (b) a Cas9 protein, or a nucleic acid encoding said protein is also provided. In one embodiment, the kit includes a vial or other vessel that contains the lentiviral vector and a pharmaceutically acceptable excipient suitable for administration to a human subject in need thereof.

In yet another aspect, the invention relates to a method of inhibiting the function or presence of a target human immunodeficiency virus 1 (HIV-1) DNA sequence in a human cell by contacting the cell, either *in vitro* or *in vivo*, with a lentiviral vector harboring: (a) one or more guide RNA, or nucleic acids encoding said one or more guide RNA; and (b) a Cas9 protein, or nucleic acids encoding said Cas9 protein, wherein the guide RNA hybridizes with the target HIV-1 DNA sequence, thereby inhibiting the function or presence of the target HIV-1 DNA sequence.

Other features and advantages of the present invention will become more readily apparent to those of ordinary skill in the art after reviewing the following detailed description and accompanying drawings.

Aspects or embodiments of the invention may also be provided according to the following paragraphs:
1. A method of suppressing HIV-1 production in a peripheral blood mononuclear cell (PBMC) of a human subject, the method comprising the steps of:
   contacting the PBMC with a composition comprising a viral vector, whereby the viral vector enters the PBMC,
   wherein the viral vector comprises nucleic acid sequences encoding a guide RNA and a humanized Cas endonuclease,
   wherein the guide RNA is complementary to a DNA sequence contained in a proviral HIV-1 genome, and
   wherein the humanized Cas endonuclease, when expressed as a protein in the PBMC, in the presence of the guide RNA cleaves within the proviral HIV-1 genome of the PBMC that contacted the viral vector, thereby suppressing HIV-1 production.
2. The method of paragraph 1, wherein the viral vector is a lentiviral vector.
3. The method of paragraph 1, wherein the humanized Cas is Cas9.
4. The method of paragraph 1, wherein the guide RNA cleaves within the proviral HIV-1 long terminal repeat (LTR).
5. The method of paragraph 1, wherein the PBMC is selected from the group consisting of a lymphocyte and a macrophage.
6. The method of paragraph 5, wherein the guide RNA cleaves within the proviral HIV-1 long terminal repeat (LTR) and the DNA sequence complementary to the guide RNA is a DNA sequence present in the proviral HIV-1 LTR, and wherein the contacting step is performed before the cell is infected with HIV-1.
7. The method of paragraph 6, wherein the DNA sequence is a U3 DNA sequence.
8. The method of paragraph 5, wherein the guide RNA cleaves within the proviral HIV-1 long terminal repeat (LTR) and the DNA sequence complementary to the guide RNA is a DNA sequence present in the proviral HIV-1 LTR, and wherein the contacting step is performed after the cell is infected with HIV-1.
9. The method of paragraph 8, wherein the DNA sequence is a U3 DNA sequence.
10. The method of paragraph 5, wherein the guide RNA cleaves within the proviral HIV-1 long terminal repeat (LTR) and the DNA sequence complementary to the guide RNA is a DNA sequence present in the proviral HIV-1 LTR, and wherein said method further comprises the step of removing the PBMC from the human subject before performing the contacting step.
11. The method of paragraph 10, wherein the DNA sequence is a U3 DNA sequence.
12. The method of paragraph 5, wherein the guide RNA cleaves within the proviral HIV-1 long terminal repeat (LTR) and the DNA sequence complementary to the guide RNA is a DNA sequence present in the proviral HIV-1 LTR, and wherein said method further comprises the step of performing the contacting step in the human subject.
13. The method of paragraph 12, wherein the DNA sequence is a U3 DNA sequence.
14. The method of paragraph 1, wherein the DNA sequence complementary to the guide RNA is a U3 DNA sequence present in the proviral HIV-1 LTR.
15. The method of paragraph 1, wherein the contacting step is performed before the cell is infected with HIV-1.
16. The method of paragraph 1, wherein the contacting step is performed after the cell is infected with HIV-1.
17. The method of paragraph 1, wherein the composition in the contacting step further comprises a pharmaceutically acceptable excipient for administration to the human subj ect.
18. The method of paragraph 1, further comprising the step of removing the PBMC from the human subject before performing the contacting step.
19. The method of paragraph 18, further comprising the step of returning the PBMC to the human subject after performing the contacting step.
20. The method of paragraph 1, wherein the viral vector is packaged within a virion that comprises an envelope protein that binds to a specific cell type.
21. The method of paragraph 2, wherein the lentiviral vector is packaged within a virion that comprises an envelope protein that binds to a specific cell type.
22. A lentiviral vector, comprising nucleic acids encoding one or more guide RNA, wherein said guide RNA hybridizes with a target HIV-1 DNA sequence, and a Clustered Regularly Interspaced Short Palindromic Repeats-Associated (Cas) protein.
23. The lentiviral vector of paragraph 22, wherein the Cas protein is a Cas9 protein.
24. The lentiviral vector of paragraph 22, wherein the lentiviral vector is g-LV.
25. A kit comprising a lentiviral vector that contains (a) one or more guide RNA, or nucleic acids encoding said one or more guide RNA, wherein said guide RNA hybridizes with a target HIV-1 DNA sequence; and (b) a Cas9 protein, or a nucleic acid encoding said protein.
26. The kit of paragraph 25, further comprising a vial or other vessel that contains the lentiviral vector and a pharmaceutically acceptable excipient suitable for administration to a human subject in need thereof.
27. A pharmaceutical composition comprising the lentiviral vector of paragraph 22.

### Brief Description of the Drawings

The structure and operation of the present invention will be understood from a review of the following detailed description and the accompanying drawings.

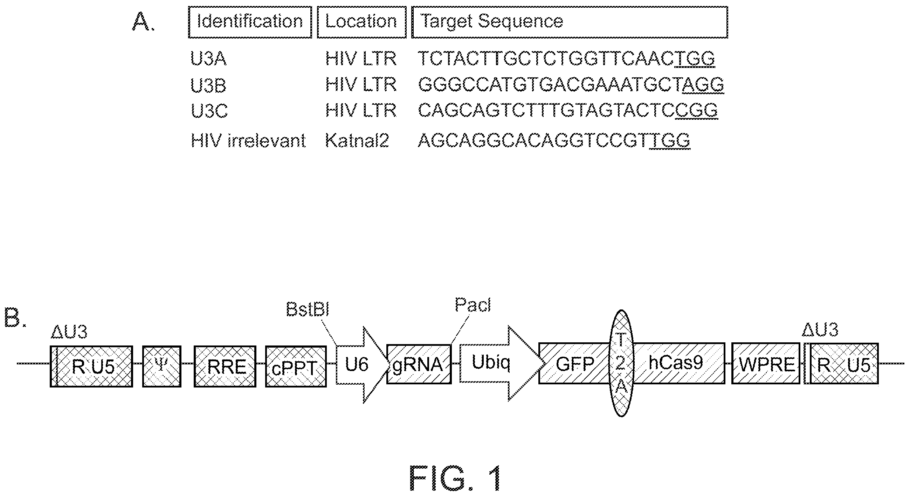

Figures 1A-1B show nucleic acid sequences and constructs used for demonstrating the invention. Figure 1A presents sequences (U3A = SEQ ID NO:6; U3B = SEQ ID NO:7; U3C = SEQ ID NO:8; and HIV irrelevant = SEQ ID NO:9) that served as targets for gRNA. Protospacer adjacent motif (PAM) sequences are underlined. Figure 1B is a schematic diagram of a lentiviral vector showing placement of sequences encoding gRNA and humanized Cas9 protein (hCas9).

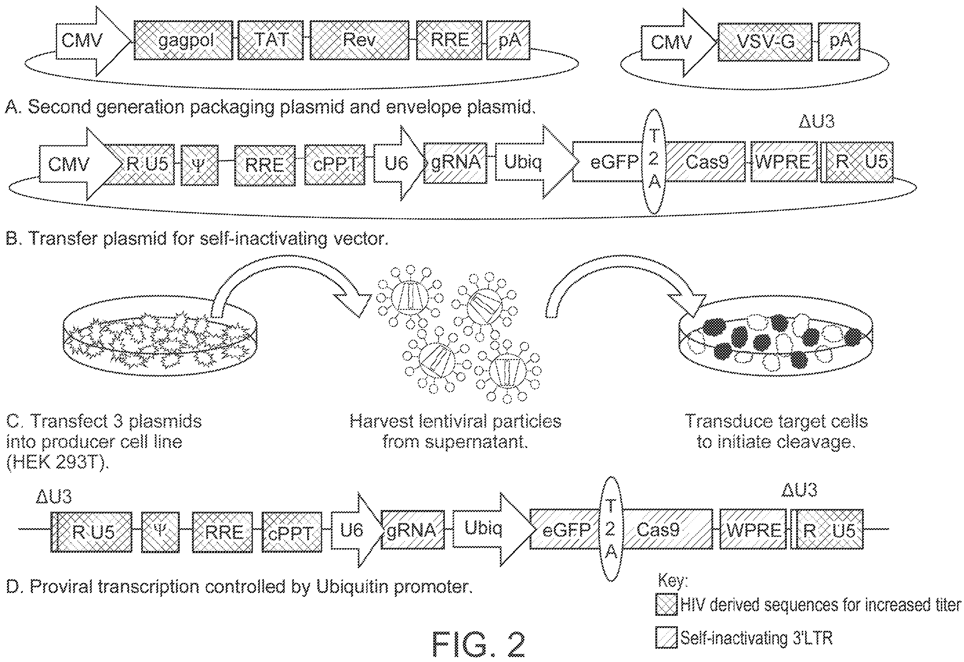

Figures 2A-2D schematically illustrates construction of a lentiviral vector for delivering gene sequences encoding HIV-1 specific gRNA and humanized Cas9 protein. Panel A shows two plasmids carrying gene sequences used for packaging and envelope production. Panel B shows a plasmid construct containing cloned sequences encoding HIV-1 specific gRNA and humanized Cas9 protein. Panel C illustrates production of the recombinant virus. Panel D illustrates the resulting integrated lentivirus construct.

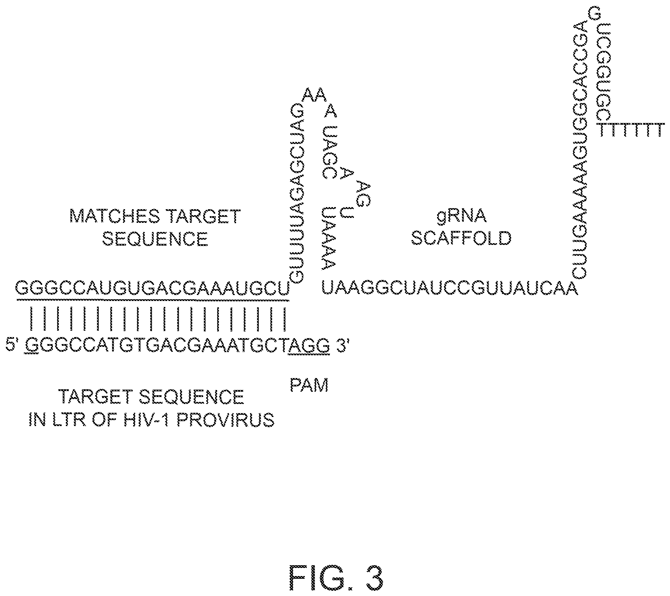

Figure 3 depicts the secondary structure of a gRNA transcript (SEQ ID NO: 10 from 5'-3') with the spacer sequence from HIV-1 LTR. SEQ ID NO:11 is the target sequence in LTR of HIV-1 provirus depicted in Figure 3.

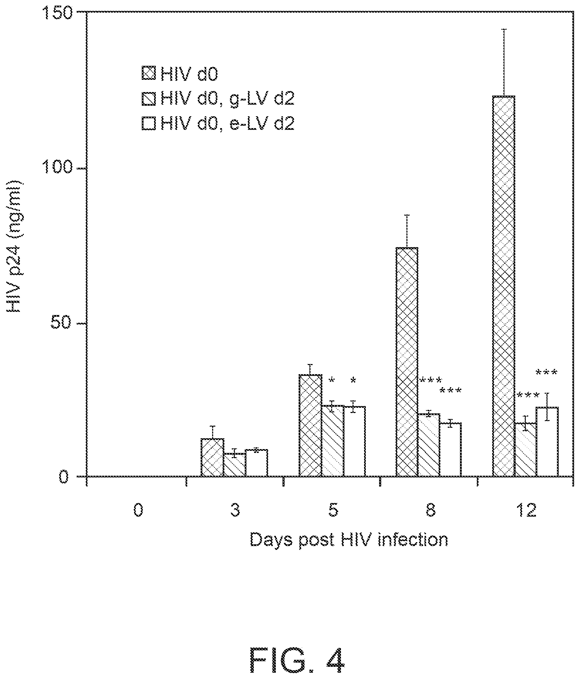

Figure 4 is a collection of bar graphs showing suppression of HIV-1 production from HIV-infected PBMC cultures transduced with lentiviral vectors expressing a transgene containing the genes for both the guide RNA and the Cas9 (g-LV), or only the Cas9 gene (e-LV). PHA activated PBMCs were infected at culture initiation (day 0) with HIV-1_{BaL} and then transduced with LV on day 2 post-HIV infection. Supernatants were collected on days 3, 5, 8 and 12 post LV transduction and HIV-1 p24 levels measured by ELISA. Suppression of HIV-1 p24 was observed in cultures transduced with either the g-LV (specific guide RNA) or e-LV (Cas9) only vectors out to day 12 post-transduction. Single asterisks (^{∗}) denote p≤0.05 and triple asterisks (^{∗∗∗}) denote p≤0.0005. Black bars indicate results from cultures that were infected with HIV-1_{BaL}, but not transduced with any recombinant lentiviral vector. Grey bars indicate results from cultures that were infected with HIV-1_{BaL} and subsequently transduced with recombinant lentiviral vector that carried an HIV-1 specific gRNA (g-LV). Open bars indicate results from cultures that were infected with HIV-1_{BaL} and subsequently transduced with the empty recombinant lentiviral vector (e-LV) that did not carry an HIV-1 specific gRNA. Results are averages of 3 separate experiments with 3 different donors, with each condition performed in triplicate.

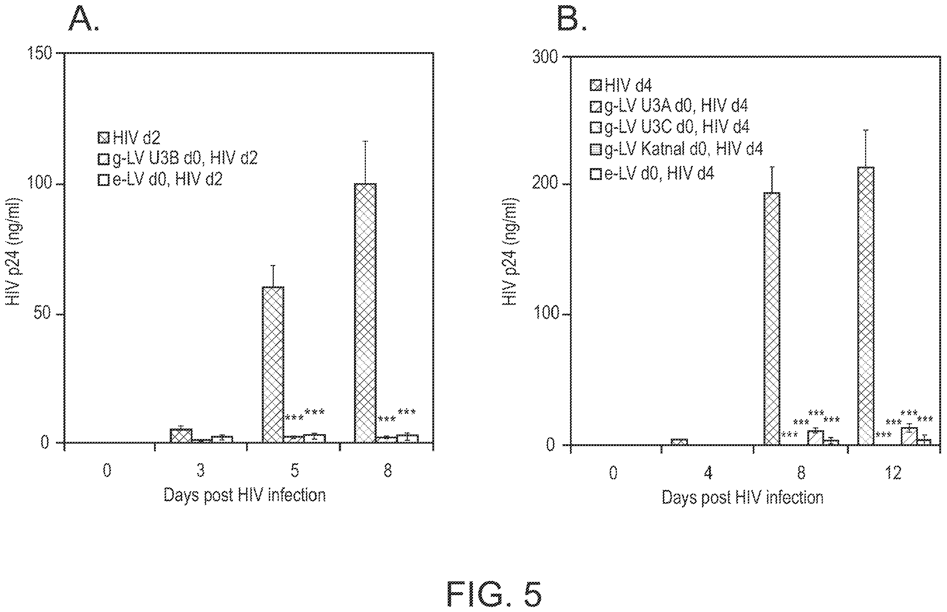

Figures 5A-5B are bar graphs providing evidence for protection of healthy PBMC from HIV-1 infection by transduction with either g-LV or e-LV. PHA activated PBMC were transduced at culture initiation (day 0) with either g-LV (specific guide RNA) or e-LV (Cas9 only) vectors, and then infected with HIV-1_{BaL} on day 2 post-LV transduction (Figure 5A) or infected with HIV-1 on day 4 (Figure 5B). Supernatants were collected on subsequent days post HIV infection and HIV-1 p24 levels measured by ELISA. Suppression of HIV-1 infection was observed with either the g-LV or e-LV vectors throughout the culture period. Triple asterisks denote p≤0.0005. Data shown are averages of three separate experiments with three different donors, with each condition performed in triplicate.

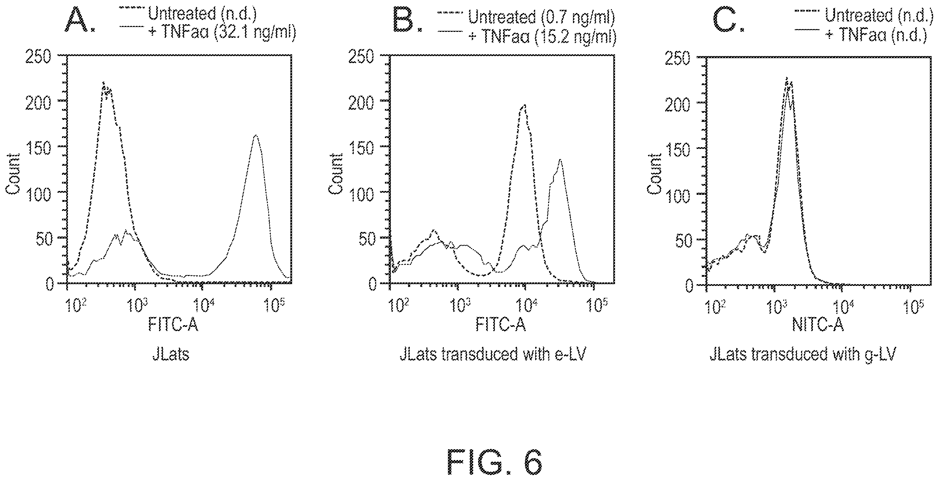

Figures 6A-6C show flow cytometric results indicating HIV-1 proviral expression is excised following transduction of J-Lat 10.6 cells by g-LV. J-Lat cells were analyzed for GFP expression from either the integrated transgene or the integrated proviral sequence by flow cytometry prior to and then after TNF-α activation. Figures 6A-6C show flow cytometric diagrams of parental cells (panel A), or selected individual clones transduced with either e-LV (panel B) or g-LV (panel C). HIV-1 p24 levels (nanogram/milliliter) were also assessed by ELISA and are noted in the legends for each histogram. "N.D." is used to indicate "not detected."

### Detailed Description

### Introduction and Overview

Provided are vectors, kits, and methods for preventing or inhibiting activity of proviral HIV-1 in a human subject. In accordance with the invention, the activity or function of an HIV-1 DNA target sequence is inhibited when the target sequence cannot be transcribed and/or translated. In this respect, the HIV-1 DNA target can be either mutated or excised. Vectors embrace lentiviral vectors adapted for delivery of components of a CRISPR/Cas9 system that directs cleavage of integrated DNA specific for portions of the HIV-1 genome. Kits include the vectors and ancillary components. Methods in accordance with the invention embrace *in vivo* and *ex vivo* techniques for introducing the vector into cells of the human subject. Use of the lentiviral vector advantageously permits transduction of cells that are dividing, as well as cells that are quiescent (*i.e.*, non-dividing). After reading this description it will become apparent to one skilled in the art how to implement the invention in various alternative embodiments and alternative applications. Although various embodiments of the present invention will be described herein, it is understood that these embodiments are presented by way of example only, and not limitation. As such, this detailed description of various alternative embodiments should not be construed to limit the scope or breadth of the present invention as set forth in the appended claims.

Certain embodiments disclosed herein provide for highly effective delivery of a CRISPR/Cas9 system using a lentiviral vector. For example, one method disclosed herein allows for lentivirus-based delivery of a guide RNA that targets sequences within the LTR of HIV-1, which, in the presence of Cas9 protein, facilitates excision of at least a portion of the HIV-1 provirus. The published PCT application identified by WO2014/165349A1, the disclosure of which is incorporated by reference, describes use of the CRISPR/Cas9 system for excising HIV-1 proviral DNA from the genome of human cells. The document does not, however, disclose use of a lentivirus as the vector for delivering nucleic acid sequences encoding Cas9 endonuclease and HIV-specific guide RNA to cells of a human.

The present disclosure provides for development of lentiviral vectors to deliver a specific gene sequence, called "a transgene," to white blood cells that are targets for HIV infection. The transgene contains two genes based on the bacterial CRISPR/Cas system. The first gene encodes the guide RNA (*i.e.*, gRNA) designed to bind to the integrated HIV gene. The second gene is the Cas9 gene that encodes an enzyme that cleaves doublestranded DNA at a region dictated by the gRNA. The guide RNA and the Cas9 enzyme function together to cleave (*i.e.*, break) the DNA within the gene that is being targeted. Lentiviral vectors can also be modified with targeting proteins, such as antibodies or other types of envelope genes, to direct the vectors to a cell of choice. Such techniques will be familiar to those having an ordinary level of skill in the art.

The approach disclosed herein employs a lentiviral delivery system to suppress viral production from HIV-infected lymphocytes using the CRISPR/Cas9 genomic engineering approach. As disclosed below, a guide RNA that targets a region in the U3 DNA sequence of the HIV-1 provirus present in both the 5' and 3' long terminal repeat (LTR) regions, together with the associated Cas9 enzyme was delivered to peripheral blood mononuclear cells (PBMC) by VSV-g pseudotyped lentiviral vectors (LVs). Cultures of PBMCs were either infected first with HIV-1, or were transduced with LV prior to infection with HIV-1. In either experimental protocol, transduction with a U3-specific guide RNA prevented the cells from producing HIV-1. Interestingly, LV expressing only the Cas9 gene had similar results, suggesting that one or more additional mechanisms, other than gene cleavage, could inhibit or prevent HIV-1 infection. Analysis of inflammatory molecules secreted by LV-transduced cells suggested that certain soluble cytokines and other inflammatory molecules induced by LV transduction could be contributing to the anti-HIV effect. Our findings indicate that CRISPR/Cas genomic engineering could be an effective therapeutic approach for use in HIV patients seeking to stop ART. In this case, LV transduction of latent HIV infected cells would cleave the provirus and prevent transcription of new virions, while LV transduction of uninfected cells would render these cells resistant to infection by HIV-1.

The disclosed procedures effectively suppressed virus production from HIV-infected PBMC, and also protected uninfected PBMC from becoming infected with HIV-1. In HIV-infected cells, the guide RNA plus Cas9 cleaves the integrated provirus, effectively preventing transcription of the structural or regulatory genes required for new virion production. In uninfected PBMCs, the guide RNA and Cas9 genes become stably integrated into the host genome where they are constitutively expressed, preventing HIV-1 cDNA integration and/or cleaving the provirus after integration. In our experimental approaches, PBMCs were infected with HIV-1 and two days later transduced with LV, or were transduced with LV first, and then challenged with HIV-1 two days later.

Results also showed that cloned populations of J-Lat cells transduced with g-LV were unable to become reactivated by TNFα to produce either GFP or p24 from the integrated proviral sequence. Subsequent analyses using PCR to amplify the proviral region in these clones showed complete excision of the proviral region. This resulted because the guide RNA to the U3 region (termed U3B) targeted a nucleic acid sequence present in both the 5' and 3' LTR. In contrast, clones of J-Lat cells transduced with the e-LV (*i.e.*, that did not encode gRNA) retained their ability to become activated with TNFα and produced both GFP and p24. J-Lat cells were used to demonstrate the excision of the proviral region because transcriptional activation by TNFα can serve as a rapid screening for competency of the provirus after CRISPR/Cas cleavage.

Also disclosed herein is the suppression of HIV-1 production from HIV-infected PBMC transduced with either the g-LV or the e-LV. This finding was unexpected, because the e-LV contains no proviral-specific gRNA sequence. This finding could have resulted from induction of innate antiviral responses induced by the e-LV. The induction of anti-viral responses following LV transduction is likely for several reasons. LV express a single-stranded RNA genome that can bind to and activate a number of innate immune receptors, including TLR7. When activated, these receptors lead to the secretion of molecules capable of protecting adjacent cells in the culture from pathogens. This could explain the finding that although only a subset of PBMC were transduced with LV (15-60% as determined by flow cytometric analysis of GFP expression), the vast majority of the cells in the population were protected from infection throughout the 12-day experimental period.

In aggregate, the results presented herein confirmed that LV delivery of the gRNA and Cas9 genes to cleave the HIV-1 provirus both protected uninfected PBMC from HIV-1 infection and also blocked HIV-1 production from infected cells. The gRNA sequence designed to the U3 region (designated U3B) in the LTR excised the entire proviral sequence from an infected cell. In addition to direct effects of the guide RNA on the proviral sequence, we also disclose that LV delivery of only the Cas9 gene (*i.e.*, not including gRNA) also protected uninfected PBMC from HIV-1, and blocked HIV-1 production from infected cells. Analysis of secreted inflammatory molecules suggests that the transgene induced immunomodulatory responses that have the potential to protect cells from HIV-1 infection.

### General Features of the CRISPR/Cas9 System

Three classes of CRISPR systems (Type I, II and III) will be familiar to those having an ordinary level of skill in the art. In certain embodiments of this invention, the Type II CRISPR system is employed because it uses a single effector enzyme, Cas9, to cleave dsDNA. In contrast, Type I and Type III systems require multiple distinct effectors acting as a complex (Makarova et al., Nat..Rev. Microbiol. 9:467 (2011)). In prokaryotes, the Type II effector system is composed of a long pre-crRNA transcribed from the spacer-containing CRISPR locus, the multifunctional Cas9 protein, and a tracrRNA for gRNA processing. The tracrRNAs hybridize to the repeat regions separating the spacers of the pre-crRNA, initiating dsRNA cleavage by endogenous RNase III, which is followed by a second cleavage event within each spacer by Cas9, producing mature crRNAs that remain associated with the tracrRNA and Cas9. However, it has been demonstrated that a tracrRNA : crRNA fusion, termed a guide RNA (gRNA) can function *in vitro* in eukaryotic cells, thereby obviating the need for RNase III and the crRNA processing in general (Jinek et al., Science 337:816 (2012)). Accordingly, in a particular embodiment of this invention, a guide RNA molecule is used to target an HIV-1 DNA sequence. Guide RNA molecules of this invention can range in size from 80 to 130 nucleotides, 90 to 120 nucleotides or 100 to 110 nucleotides in length and are composed of RNA. In particular embodiments, the guide RNA has the sequence R-GU UUU AGA GCU AGA AAU AGC AAG UUA AAA UAA GGC UAG UCC GUU AUC AAC UUG AAA AAG UGG CAC CGA GUC GGU GCT TTT TT (SEQ ID NO:1), wherein R is a spacer sequence that specifically hybridizes to an HIV-1 DNA target sequence.

In prokaryotes, Type II CRISPR interference is a result of Cas9 unwinding the DNA duplex and searching for sequences matching the crRNA to cleave. Target recognition occurs upon detection of complementarity between a "protospacer" sequence in the target DNA and the remaining spacer sequence in the crRNA. Cas9 cuts the DNA only if a correct protospacer-adjacent motif (PAM) is also present at the 3' end. Different Type II systems have differing PAM requirements. The *S. pyogenes* system requires an NGG sequence, where N can be any nucleotide (Mali et al., Science 339:823-6 (2013)). *S. thermophilus* Type II systems require NGGNG (Horvath et al., Science 327:167 (2010)) and NAGAAW (Deveau et al., J. Bacteriol. 190:1390 (2008)), respectively, while different *S. mutans* systems tolerate NGG or NAAR (van der Ploeg et al., Microbiology 155:1966 (2009)).

In particular embodiments of the invention, the gRNA targets the LTR portion of the HIV-1 DNA. The LTR functions to promote and regulate the transcription of the genes that are essential for assembling new virions, as well as transcribing the entire HIV-1 genome, which is packaged into infectious particles. There are two copies of the LTR in the integrated provirus, thereby doubling the size of the instant target. The above identified LTR target sequences have the required PAM sequence and are unique to HIV-1, having no cross-reacting homologies in the human genome. This is of particular importance because the human genome contains many other retroviruses with LTR- like sequences. Thus, very low or no cytotoxicity is expected in uninfected cells.

The Type II CRISPR systems include the HNH-type system (Streptococcus-like; also known as the Nmeni subtype, for *Neisseria meningitidis* serogroup A str. Z2491, or CASS4), in which Cas9 is sufficient for generating crRNA and cleaving the target DNA. Cas9 contains at least two nuclease domains, a RuvC-like nuclease domain near the amino terminus and the HNH (or McrA-like) nuclease domain in the middle of the protein. Given that the HNH nuclease domain is abundant in restriction enzymes and possesses endonuclease activity (Kleanthous et al., Nat. Struct. Biol. 6:243-52 (1999); Jakubauskas et al., J. Mol. Biol. 370:157-169 (2007)), it has been suggested that this domain is responsible for target cleavage. Furthermore, for the *S. thermophilus* type II CRISPR-Cas system, targeting of plasmid and phage DNA has been demonstrated *in vivo* (Garneau et al., Nature 468:67-71 (2010)) and inactivation of Cas9 has been shown to abolish interference (Barrangou et al, Science 315:1709-12 (2007)). In some embodiments of this invention, the Cas protein used in the cleavage of the HIV-1 target sequence is a Type II Cas protein. In other embodiments, the Cas protein is a Type II Cas protein isolated from *Streptococcus thermophilus*, *Listeria innocua*, *Neisseria meningitidis* or *S. pyogenes*. In other embodiments, the Cas protein is a Cas9 protein isolated from *Streptococcus thermophilus* (*see* GENBANK Accession No. YP_820832), *Listeria innocua* (*see* GENBANK Accession No. NP_472073), *Neisseria meningitidis* (*see* GENBANK Accession No. YP_002342100) or *S. pyogenes* (*see* GENBANK Accession Nos. AAK33936 and NP_269215). In addition to these species variants the Cas9 protein may harbor engineered mutations improving specificity or efficacy (Slaymaker et al., Science 351:84-88 (2016)). In certain embodiments, the Cas9 protein has been codon-optimized for expression in human. Plasmids harboring nucleic acids encoding Cas9 proteins are available from repository sources such as Addgene (Cambridge, MA). Examples of such plasmids include pMJ806 (*S. pyogenes* Cas9), pMJ823 (*L. innocua* Cas9), pMJ824 (*S*. *thermophilus* Cas9), pMJ839 (*N. meningitides* Cas9), and pMJ920 (human codon-optimized Cas9).

To facilitate entry into the nucleus, the Cas protein of the invention can include a nuclear localization sequence (NLS). The term "nuclear localization sequence" means an amino acid sequence that induces transport of molecules having such sequences or linked to such sequences into the nucleus of eukaryotic cells. In this context, the term "having" preferably means that the nuclear localization sequence forms part of the molecule, *i.e.* that it is linked to the remaining parts of the molecule by covalent bonds, *e.g.*, an in-frame protein fusion. The term "linked" in this context means any possible linkage between the nuclear localization sequence and another molecule to be introduced into the nucleus of a eukaryotic cell, for example by covalent bonds, hydrogen bonds or ionic interactions. The term "transport into the nucleus" in this context means that the molecule is translocated into the nucleus.

Nuclear translocation can be detected by direct and indirect means. For example, direct observation can be achieved by fluorescence or confocal laser scanning microscopy when the nuclear localization sequence or the translocated molecule (*e.g.*, the Cas protein or guide RNA) are labeled with a fluorescent dye (labeling kits are commercially available, for example, from Pierce or Molecular Probes). Translocation can also be assessed by electron microscopy if the nuclear localization sequence or the translocated molecule (*e.g.*, the Cas protein or guide RNA) is labeled with an electron-dense material such as colloidal gold (Oliver MethodsMol. Biol. 115:341-345 (1999)). Translocation can be assessed in indirect ways, such as by measuring cleavage of a target HIV-1 DNAs sequence.

A number of nuclear localization sequences have been described in the art, and may be used in connection with the present invention. Those having an ordinary level of skill in the art will appreciate that numerous exemplary nuclear localization sequences have been set forth by Howell et al., in WO2014/165349, the disclosure of which is incorporated by reference.

### Useful Lentivirus Vectors

Lentiviral vectors of the present invention share certain advantages over different vectors that may have been used previously. For example, recombinant retrovirus vectors based on MoMuLV (moloney murine leukemia virus) can only infect dividing cells because the virus does not have a mechanism to move its genome through the nuclear envelope. Thus, the viral genome only has access to the host cell genome when the nuclear envelope is naturally broken down during mitosis. The recombinant lentivirus employed in the procedures described herein, based on HIV-1, is capable of transporting its genome through the nuclear envelope of the host cell. The recombinant lentivirus vector is distinct from the retroviral vectors in the sequences for the LTRs and the Psi gene. Further the packaging of the lentivirus is distinct from the retrovirus because the sequence of the Gag and Pol genes in the packaging plasmid is distinct in the two systems. However, the internal elements driving expression of the guide RNA and the Cas9 can be identical between lentiviral and retroviral systems. This allows for interchanging between the two systems to make equivalent lentivirus or retrovirus depending on whether the procedure calls for a need to specifically infect dividing cells only. In some instances, the retroviral system is preferred as a therapeutic for cancer where targeting dividing cells could provide specificity towards the cancer. The base vector for the lentivirus called FUGW (available from the nonprofit plasmid repository ADDGENE as plasmid # 14883; Hong et al., Science. 2002 Feb 1. 295(5556):868-72) will be familiar to those having an ordinary level of skill in the art. Modifications made in the course of developing the present invention included the insertion of GFP-T2A-Cas9 driven by the ubiquitin promoter (see, e.g., Williams et al., Sci. Rep. 6, 25611; doi: 10.1038/srep25611 (2016); incorporated herein by reference in its entirety), and the use of HIV-1 specific guide RNAs driven by the U6 promoter.

In addition to the FUGW base lentiviral vector described above, other suitable lentiviral vectors contemplated herein for use in the present invention may be derived from or may be derivable from any suitable lentivirus (see US 9,339,512; which is incorporated herein by reference in its entirety). A recombinant lentiviral particle is capable of transducing a target cell with a nucleotide of interest (NOI), such as the guide RNA and CAS9 genes described herein, and the like. Once within the cell the RNA genome from the vector particle is reverse transcribed into DNA and integrated into the genome of the target cell.

Lentiviral vectors are part of a larger group of retroviral vectors. A detailed list of lentiviruses may be found in Coffin et al. (1997) "Retroviruses" Cold Spring Harbor Laboratory Press Eds: J M Coffin, S M Hughes, HE Varmus pp 758-763). In brief, lentiviruses can be divided into primate and non-primate groups. Examples of primate lentiviruses include but are not limited to: the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

Lentiviruses differ from other members of the retrovirus family in that lentiviruses have the capability to infect both dividing and non-dividing cells (Lewis et al (1992) EMBO J 11(8):3053-3058) and Lewis and Emerman (1994) J Virol 68 (1):510-516). In contrast, other retroviruses--such as MLV--are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The basic structure of retrovirus and lentivirus genomes share many common features such as a 5' LTR and a 3' LTR, between or within which are located a packaging signal to enable the genome to be packaged, a primer binding site, integration sites to enable integration into a host cell genome and gag, pol and env genes encoding the packaging components--these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, the viral genes are flanked at both ends by regions called long terminal repeats (LTRs). The LTRs are responsible for proviral integration, and transcription. LTRs also serve as enhancer-promoter sequences and can control the expression of the viral genes.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different viruses.

In a defective lentiviral vector genome gag, pol and env may be absent or not functional. The R regions at both ends of the RNA are repeated sequences. U5 and U3 represent unique sequences at the 5' and 3' ends of the RNA genome respectively.

In a typical lentiviral vector of the present invention, at least part of one or more protein coding regions essential for replication may be removed from the virus. This makes the viral vector replication-defective. Portions of the viral genome may also be replaced by an NOI in order to generate a vector comprising an NOI (such as the guide RNS and CAS9 genes described herein and set forth in g-LV described herein) which is capable of transducing a target non-dividing host cell and/or integrating its genome into a host genome.

In one embodiment the lentiviral vectors are non-integrating vectors as described in WO 2007/071994. In a further embodiment the vectors have the ability to deliver a sequence which is devoid of or lacking viral RNA. In a further embodiment a heterologous binding domain (heterologous to gag) located on the RNA to be delivered and a cognate binding domain on gag or pol can be used to ensure packaging of the RNA to be delivered. Both of these vectors are described in WO 2007/072056.

The lentiviral vector may be a "non-primate" vector, i.e., derived from a virus which does not primarily infect primates, especially humans. The examples of non-primate lentivirus may be any member of the family of lentiviridae which does not naturally infect a primate and may include a feline immunodeficiency virus (FIV), a bovine immunodeficiency virus (BIV), a caprine arthritis encephalitis virus (CAEV), a Maedi visna virus (MVV) or an equine infectious anaemia virus (EIAV).

In some optional embodiments, vectors of the present invention are recombinant lentiviral vectors. The term "recombinant lentiviral vector" refers to a vector with sufficient lentiviral genetic information to allow packaging of an RNA genome, in the presence of packaging components, into a viral particle capable of infecting a target cell. Infection of the target cell may include reverse transcription and integration into the target cell genome. The recombinant lentiviral vector carries non-viral coding sequences which are to be delivered by the vector to the target cell. A recombinant lentiviral vector is incapable of independent replication to produce infectious lentiviral particles within the final target cell. Usually the recombinant lentiviral vector lacks a functional gag-pol and/or env gene and/or other genes essential for replication. The vector of the present invention may be configured as a split-intron vector. A split intron vector is described in PCT patent application WO 99/15683.

Optionally, the recombinant lentiviral vector of the present invention has a minimal viral genome. As used herein, the term "minimal viral genome" means that the viral vector has been manipulated so as to remove the non-essential elements and to retain the essential elements in order to provide the required functionality to infect, transduce and deliver a nucleotide sequence of interest to a target host cell. Further details of this strategy can be found in our WO 98/17815.

Figures 1A and 1B respectively illustrate DNA targets for example gRNAs, and the structure of an exemplary lentiviral vector useful for transducing gRNA and humanized Cas9 sequences. Figure 1A presents DNA target sequences used for designing gRNAs (shown 5' to 3') with the protospacer adjacent motif (PAM) sequences underlined. Guide RNA sequences to the HIV-1 proviral LTR U3 region, designated U3A, U3B, and U3C, are listed. In addition, a target sequence to the Katnal 2 gene was used as an irrelevant gRNA for secreted cytokine experiments. Figure 1B schematically depicts the integrated g-LV transgene showing the gene regions derived from HIV-1; 5' LTR (grey boxes) with the deleted U3 region (ΔU3), regulatory (R) and U5 regions, packaging signal (Ψ), rev response element (RRE), and central polypurine tract (cPPT, Flap element). Promoters are depicted as white arrows: U6 is the mammalian promoter for U6 small nuclear RNAs, and Ubiq is the human ubiquitin-c promoter. The transcriptional products are shown as heavy lined boxes; gRNA is the guide RNA that targets DNA sequences in the genome, GFP encodes the green fluorescent protein, T2A is the self-cleaving peptide, and hCas9 is the humanized Cas9 endonuclease. WPRE is the woodchuck hepatitis virus post-transcriptional regulatory element. The 3' LTR also had the U3 region deleted (ΔU3) upstream of the regulatory and U5 region.

Lentiviral vectors useful in connection with the disclosed procedure were constructed by co-transfecting three different nucleic acid constructs into a packaging cell line (*see* Figure 2C. More particularly, these constructs (depicted in Figures 2A and 2B) were transfected into a packaging cell line, such as HEK293FT cells. The packaging cells transcribed and translated the proteins in the packaging and envelope plasmids (depicted in Figure 2A) and produced lentivirus vectors that were secreted into the media of the cells. The construct illustrated in Figure 2B is a transgene carrying the guide RNA and humanized Cas9 genes. Media from co-transfected cells was collected, concentrated, titered, and used to transduce the transgene (shown in Figure 2B) into the cell of choice. Specificity of targeting of the lentivirus vector to cells of choice can be increased by including genes for antibodies or ligands directed to cell surface receptors or by replacing the envelope gene with another that provides cell specificity of binding. These antibody, ligand, or envelope genes are transfected into the HEK293FT cells at the same time as the packaging, envelope and transfer plasmids.

The lentivirus produced by this approach can then be added to target cells, such as human macrophages and T cells. The envelope proteins on the lentivirus bind to specific or non-specific receptors on the surface of the target cell and are internalized via endosomes into the target cell. There, the transgene (carrying the guide RNA and Cas9 genes) is translocated to the nucleus where it is integrated into the genome. Once integrated, the guide RNA sequence is transcribed from the U6 promoter, and the Cas9 and optional green fluorescent protein (GFP) genes are transcribed from the ubiquitin promoter and then translated into functional proteins. The Cas9 protein advantageously has a nuclear localization sequence that directs it to the nucleus where it functions together with the guide RNA to bind to and cleave a specific region in the human genome.

### Sequences Encoding Guide RNAs

Figure 3 shows an example of a guide RNA (top sequence of nucleotides) designed for the target DNA sequence of the HIV-1 provirus that has a PAM sequence (bottom sequence of nucleotides; PAM sequence is underlined). As shown, these two sequences match. The guide RNA, however, will bind to the complementary strand of the target DNA sequence (not shown in this figure).

### Statistical Analysis

Analyses of data from PBMC p24 production following HIV-1 infection were performed by ANOVA. Data were considered statistically significant when the student's t-test gave p ≤ 0.05. Specific p values are noted in each figure legend. The data are represented as the mean ± standard error of the mean of experiments from either of three or four different blood cell donors, with each experimental condition assayed in triplicate.

### Pharmaceutical Compositions

The lentiviral vectors of the present invention may be provided in the form of a pharmaceutical composition. The pharmaceutical composition may be used for treating an individual by gene therapy, wherein the composition comprises a therapeutically effective amount of a particular lentiviral vector.

The viral preparation may concentrated by ultracentrifugation. WO 2009/153563 describes methods for the downstream processing of lentiviral vectors. The resulting pharmaceutical composition may have at least 10⁷ T.U./mL, for example from 10⁷ to 10⁹ T.U./mL, or at least 10⁹ T.U./mL. (The titer is expressed in transducing units per mL (T.U./mL) as titred on a standard D17 of HEK293T cell lines).

The pharmaceutical composition may be used to treat a human or animal, for example a primate animal subject or a companion animal subject.

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as (or in addition to) the carrier, excipient or diluent, any suitable binder(s), lubricant(s), suspending agent(s), coating agent(s), solubilising agent(s), and other carrier agents that may aid or increase the viral entry into the target site (such as for example a lipid delivery system).

### Methods of Treatment and/or Prevention

An invention lentivral vector, such as "g-LV" and the like, can be used to treat HIV infected individuals, delay the onset of AIDS and treat AIDS patients, both in vivo and ex vivo. In another embodiment, an invention lentiviral vector can be used to prevent HIV infection by administrating to a healthy individual.

In one embodiment, an in vivo method is provided for treating or preventing HIV infection. A subject is administered an invention lentiviral vector such as g-LV or the like. The lentiviral vector can be packaged with pharmaceutically suitable salts and solvates, administered to an individual infected with HIV virus or an AIDS patient.

An invention lentiviral vector may be conveniently administered in unit dosage form, and may be prepared by any of the methods well known in the pharmaceutical art. In some embodiments, the gene therapy lentiviral agent further comprises at least one other common excipient for treating HIV infection, such as sterile water or saline, polyalkylene glycols, oils of vegetable origin, hydrogenated naphtalenes, and the like.

An invention lentiviral vector may be formulated into pharmaceutical compositions by admixture with pharmaceutically acceptable non-toxic excipients or carriers, such as salts and solvates. Such compounds and compositions may be prepared in the intravenous form, subcutaneous form, or intramuscular form for parenteral administration, particularly in the form of liquid solutions or suspensions in aqueous physiological buffer solutions; for oral administration, particularly in the form of tablets or capsules; or in inhalation form for intranasal administration, particularly in the form of powders, nasal drops, or aerosols; or on the mucosa forms of liquid solutions or suspensions for applications. Sustained release compositions are also encompassed by the present invention. Compositions for other routes of administration may be prepared as desired using standard methods.

In other embodiments, the invention also relates to an article of manufacture containing at least one packaging material and an invention lentiviral vector, such as g-LV or the like, contained within the packaging material. The packaging material may contain a label or package insert indicating that the invention lentiviral vector may be used for treating HIV infection and AIDS.

As set forth herein, both in vivo and ex vivo methods are contemplated herein, which methods are particularly useful for treating HIV infected individuals, delaying the onset of AIDS and treating AIDS patients. In one embodiment, an ex vivo method is particularly useful for treating HIV infected individuals, delaying the onset of AIDS and treating AIDS patients, and utilizes the ex vivo transformation of bone marrow stem cells. In this particular embodiment, standard clinical procedures to draw bone marrow from the patient are used. The advantage of this particular ex vivo procedure is that it targets mainly the hematopoietic stem cells that will replicate and differentiate to all types of blood cell including T cells and macrophages.

In one embodiment, the ex vivo procedure optionally utilizes withdrawing bone marrow from the HIV-infected individual. The hematopoietic stem cells from the bone marrow are isolated and cultivated using methods known to the art, and transformed with a therapeutically effective amount of an invention lentiviral vector in a pharmaceutical composition. The transformed hematopoietic stem cells are then verified to confirm the disruption, eradication and/or elimination of the HIV proviral sequence as described herein. After screening and selecting, the transfected hematopoietic stem cells are transplanted back into the HIV-infected individual.

In another embodiment of an ex vivo procedure for use with and invention lentiviral vector composition, such as g-LV and like, PBMCs, T cells and/or macrophages are isolated from the HIV infected individual or the AIDS patient, and then the isolated cells are transformed with an invention lentiviral vector composition. This particular ex vivo procedure requires withdrawing blood from the HIV-infected individual, then the PBMC cells, T cells and/or macrophages are isolated from the blood, cultivated and transformed with a therapeutically effective amount of an invention lentiviral vector as set forth herein. The transformed PBMCs, T cells and/or macrophages are then verified to confirm the disruption, eradication and/or elimination of the HIV proviral sequence as described herein. After screening and selecting, the transformed PBMCs, T cells and/or macrophages are transfused back into the HIV-infected individual.

### Working Examples

The present invention embraces treatment of HIV-infected patients with lentiviral vectors containing gRNA and Cas9 genes. The gRNA should be specific for the HIV provirus. In this respect, the invention embraces transducing either uninfected cells or HIV infected cells with lentiviral vectors carrying HIV-specific transgenes. Example 8 addresses viral transduction of cells already infected with HIV-1. Example 9 addresses viral transduction that precedes infection with HIV-1. This therapeutic intervention would be administered to patients with HIV or patients at high risk for getting HIV before, during or after antiretroviral therapy. In one embodiment, therapeutic intervention is administered while their viremia is suppressed, either because they are "elite controllers," or because they are receiving, or have received, antiretroviral therapy. In one embodiment, this therapeutic intervention could be administered as a preventative therapeutic "vaccine" to prevent HIV in high risk individuals. Patients will be treated when their circulating immune cells are largely uninfected. After one or more infusions with lentiviral vectors, antiretroviral therapy can be withdrawn. By the approach disclosed herein, cells transduced with the HIV provirus-specific guide RNA will be protected from HIV infection from ensuing viral resurgence.

### Example 1

### Methods of Isolating and Activating Peripheral Blood Cells

Peripheral blood mononuclear cells (PBMCs) were obtained from whole blood of normal human donors. Whole blood was diluted with equal parts normal saline, layered over a cushion of Ficoll-Hypaque (Amersham, Piscataway, NJ), and centrifuged at 400x g for 30 minutes. The PBMC interface was collected, washed three times, and diluted to 2 × 10⁶ cells/ml in RPMI 1640 medium (Corning Cellgrow, Manassas, VA) supplemented with 2 mM glutamine, penicillin/streptomycin, and 10% fetal bovine serum (Atlanta Biologicals, Atlanta, GA) (cRPMI). Lymphocytes in the PBMC fraction were activated by the addition of 2 µg/ml phytohemaglutinin P (Sigma Chemical Company; St. Louis, MO) for 48 hours. Cells were then washed three times in serum-free RPMI 1640 and adjusted to 1 × 10⁶ cells/ml in cRPMI.

Each donor was pre-screened for absence of the delta 32 mutation (Δ32) in CCR5. Briefly, genomic DNA was isolated from 1-2 × 10⁶ PBMC by lysis in Qiagen AL buffer, treated with protease, and purified on a QIAmp column as directed by the manufacturer. Approximately 200 nanograms of purified DNA was subject to PCR utilizing the forward primer: 5'-GATAGGTACCTGGCTGTCGTCCAT-3' (SEQ ID NO:2), and reverse primer: 5'-ACCAGCCCCAAGATGACTATCT-3' (SEQ ID NO:3). Homozygous wild type CCR5 gave 239 base pair (bp) product, whereas the Δ32 mutation produced a 207 bp product. Donors heterozygous for this mutation expressed both the 239 and 207 bp products. Only donors homozygous for wild type CCR5 were used for this study.

### Example 2

### Methods of Identifying Guide RNA Sequences

Candidate gRNA target sequences in the HIV-1 provirus were identified using Gene Construction Kit software (Textco Biosoftware, West Lebanon, NH), and tested for unintended homologies to human genomic DNA using the BLAST program from the National Center for Biotechnology Information. These sequences were cloned by the Gibson method (available as a kit from New England Biolabs) into the Addgene vector containing the guide RNA scaffold under transcriptional control of the human U6 promoter (Addgene plasmid # 41824). Antibiotic resistant transformants were confirmed by DNA sequencing. Guide RNAs were tested for efficacy of cleavage when co-transfected with the plasmid carrying the humanized Cas9 gene (Addgene plasmid 41815) by transient transfection utilizing X-tremeGENE (Roche #06 366) as the transfection agent into J-Lat 10.6 cells. Loss of induction of J-Lat proviral GFP expression following 10 ng/ml TNFα (R&D Systems; Minneapolis, MN) activation indicated specificity of the guide sequence to the provirus. The guide RNA used in these studies targeted a region in U3 (termed "U3B") present in both the 5' and 3' LTR regions. In addition, guide RNAs to two other regions in U3 were identified ("U3A" and "U3C"). Target sequences for these guide RNAs are shown in Figure 1A. Figure 3 shows how an exemplary gRNA (top sequence of nucleotides) binds in a complementary way to the target gene region.

### Example 3

### Methods of Cloning Guide RNA and Cas9 into a Lentiviral Vector

Specific gRNA sequences chosen for cloning into the transfer vector were selected based on their ability to block GFP induction from the integrated provirus in the J-Lat 10.6 indicator cells after TNFα activation. Based on this screening, we cloned three gRNAs (termed U3A, U3B and U3C) into a self-inactivating lentiviral transfer vector derived from FUGW. The FUGW vector is based on HIV-1 and contains the HIV-1 Flap element, the human ubiquitin-c promoter, GFP, and the woodchuck hepatitis virus post-transcriptional regulatory element (WRE). Humanized Cas9 (from AddGene pX330) was cloned into this transfer vector such that it is linked to GFP via the self-cleaving peptide, T2A, and was under transcriptional control of the ubiquitin-c promoter. This transfer vector was further altered to introduce PacI and BstB1 restriction endonuclease cleavage sites adjacent to the Flap element, thereby allowing the cloning of PCR products that encompass the U6 promoter and target sequences and gRNA scaffold (Figure 1B). Our control LV was designed to express only the GFP-Cas9 gene (termed "empty LV," or "e-LV").

### Example 4

### Methods of Producing Lentivirus

Lentiviral packaging was performed by calcium-phosphate-mediated transfection of HEK293 FT cells (Invitrogen; Grand Island, NY) with three plasmids: pCMVdelta8.9, pVSV-g, and the FUGW-based transfer vector, by methods that will be familiar to those having an ordinary level of skill in the art. Briefly, cells were maintained in Iscove's Modified Dulbecco's Medium (IMDM, high glucose) (GIBCO 12440-053), 10% fetal bovine serum (FBS), 0.1 mM Non-Essential Amino Acids, 2 mM L-glutamine, 1% Pen-Strep, and 500 µg/ml geneticin. To optimize cell growth, these cells were trypsinized and replated at 2.5 - 3.0 × 10⁶ cells per 10 cm plate the day before transfection, and transfected in the absence of geneticin with approximately 10 µg transfer vector, 6.5 µg pCMVdelta8.9, and 4.5 µg pVSV-g per 10 cm plate. Twenty-four hours after transfection, the media was replaced with IMDM plus 0.5% FBS and 0.4 mg/ml caffeine. Supernatant containing lentiviral particles was collected at 48 and 72 hours post transfection, centrifuged to remove cell debris, and filtered through a 0.45 µm PVDF filter. Viral particles were concentrated by adding sterile polyethylene glycol 6000 and sodium chloride to final concentrations of 8% and 0.3 M, respectively, and incubating at 4°C for twelve or more hours followed by centrifugation at 2,500 × g for 45 minutes. The supernatant was thoroughly removed and the pellet dissolved in sterile phosphate buffered saline. The lentivirus was titered on HEK293 FT cells by serial dilution and fluorescent microscopic inspection for GFP expression 72 hours later. Titers were routinely between 10⁷ and 10⁹ transducing units/ml. Concentrated LV was stored in aliquots of 5-10 µl at - 80°C. Preparation of lentiviral vectors will also use the "third-generation" lentiviral vector system in which three plasmids, in addition to the transfer vector are used. The first plasmid will have the gag and pol genes and is called pMDLg/pRRE (Addgene plasmid # 12251), and the second plasmid will have the rev gene and is called pRSV-Rev (Addgene plasmid #12253). The third plasmid, containing the envelope gene, will express the VSV-g gene (Addgene pMD2.G, #12259).

### Example 5

### Methods of HIV-1 Infection and LV Transduction of PBMCs

Activated PBMCs were infected with HIV-1_{BaL} at a concentration of 125 TCID₅₀/10⁶ cells for 5 hours before washing out unincorporated virus after 5 hours. Cells were washed three times and placed in cRPMI containing 10 units/ml of interleukin 2 (IL-2) (Invitrogen; Carlsbad, CA).

Activated PBMC were transduced either before or after HIV-1 infection with purified LV expressing an HIV-specific guide RNA plus Cas9-GFP (g-LV) or Cas9-GFP only (e-LV). At the time of cell transduction, aliquots of LV were thawed on ice, spun in a microfuge for 5 seconds, and immediately added to PBMC plated in wells of a 24-well plate in 1 ml cRPMI. The plates were gently swirled to distribute the lentiviral vector, and then placed in a 37°C incubator with 5%CO₂ for 5 hours prior to washing the cells. PBMCs were maintained in cRPMI containing 10 units/ml IL-2 after infection with HIV-1 or transduction with LV. We quantified HIV-1 p24 levels in culture supernatant on subsequent days by ELISA using an HIV-1 p24 kit (Perkin Elmer; Waltham, MA).

### Example 6

### Method of Demonstrating Proviral Cleavage by g-LV

To confirm cleavage and/or excision of the integrated provirus by specific guide RNA sequences, J-Lat 10.6 cells were transduced with either the g-LV or the e-LV vectors, and then subjected the cell population to limiting dilution cloning. Clones that were transduced with LV expressed GFP constitutively from the GFP-Cas9 region of the transgene at moderate levels of mean fluorescence intensity (MFI) levels. GFP-positive clones were expanded, activated for 24 hours with 10 ng/ml TNFα, and then assessed for de novo GFP expression from the integrated provirus. GFP expression from the integrated provirus had a brighter MFI and fluorescence peak was clearly distinguishable from the constitutive GFP production from the integrated transgene. ELISA was used to measure levels of secreted p24.

The J-Lat clones were subjected to PCR analyses to specifically detect the vpr-tat region (176 base pairs) of the HIV provirus with the forward primer: GGCGTTACTCGACAGAGGAG (SEQ ID NO:4) and the reverse primer: TCCTGCCATAGGAGATGCCT (SEQ ID NO:5). Vpr-tat sequences are 5.5 Kb and 3.4 Kb from the U3 region in the 5' and 3' LTR, respectively. As an internal control, PCR of the CCR5 gene sequences (239 base pairs, primers listed above) were used to confirm equal template loading. PCR products from both the g-LV and e-LV clones were analyzed by agarose gel electrophoresis and visualized by staining with GelRed nucleic acid stain (Biotium; Hayward, CA).

### Example 7

### Method of Performing Multiplex Cytokine Assays

Cytokines secreted into the culture media after HIV-1 infection or LV transduction were measured using Millipore human cytokine multiplex kits (EMD Millipore Corporation; Billerica, MA). Calibration curves from recombinant cytokine standards were prepared with threefold dilution steps in the same media as the samples. High and low spikes (supernatants from stimulated human PBMCs and dendritic cells) were included to determine cytokine recovery. Standards and spikes were measured in triplicate, samples were measured once, and blank values were subtracted from all readings. All assays were carried out directly in a 96-well filtration plate (Millipore; Billerica, MA) at room temperature with protection from light.

Briefly, wells were pre-wet with 100 µl PBS containing 1% BSA, then beads, standards, samples, spikes, or blanks were added in a final volume of 100 µl and incubated together at room temperature for 30 minutes with continuous shaking. Beads were washed three times with 100 µl PBS containing 1% BSA and 0.5% Tween 20. A cocktail of biotinylated antibodies (50 µl/well) was added to the beads for a further 30 min incubation with continuous shaking. Beads were washed three times, and streptavidin-PE was then added for 10 min. Beads were again washed three times and suspended in 125 µl PBS containing 1% BSA and 0.5% Tween 20. The fluorescence intensity of the beads was measured using the Bio-Plex array reader. Bio-Plex Manager software with five parametric curve fitting was used for data analysis.

### Example 8

### LV Transduction of HIV-1 Infected PBMC Blocks HIV-1 Production

Activated PBMC were infected with HIV-1BaL 48 hrs before transduction with LV containing the U3B guide RNA and Cas9 (g-LV), or LV expressing only the GFP-Cas9 gene (e-LV). Supernatants from each of these cell cultures were collected on various days post-transduction and the concentration of p24 in the supernatant measured by ELISA as an indication of HIV-1 production. Steady increases in p24 levels were observed in those wells that were infected with HIV alone throughout the 12 days after infection, as shown in Figure 4. However, cultures that were transduced with either g-LV or e-LV demonstrated suppression of HIV p24 production throughout the 12-day period. Data presented in Figure 4 is a compilation of three different experiments, with each experimental condition performed in triplicate.

### Example 9

### Transduction of g-LV or e-LV Prior to HIV-1 Exposure Protects PBMC from Infection

PHA-activated PBMC were transduced with g-LV or e-LV to assess whether pre-integration of the guide RNA and Cas9 sequences would protect PBMC from HIV-1 infection. All cultures were infected with HIV-1 at two days post-transfection. In these cultures, both the g-LV and e-LV transduced cells were resistant to a subsequent HIV-1 infection. Data presented in Figure 5A is a compilation of three different experiments, with each experimental condition performed in triplicate.

In separate experiments, we challenged e-LV or g-LV transduced cells to HIV-1 on days 3 and 4 post-LV transduction and showed that these cells were also protected from HIV-1 infection. Results from this procedure are presented in Figure 5B.

### Example 10

### Inflammatory Chemokines Produced by LV-Transduced PBMC

To investigate alternative mechanisms by which PBMC would be able to resist infection with HIV after LV transduction, supernatant were analyzed using Luminex multiplex assays for forty-one different inflammatory cytokines from PBMC at various times after infection with HIV-1, or after transduction with g-LV, e-LV, or an LV that expresses neither a guide RNA or Cas9 in the transgene (FUGW). The g-LV included those with guide RNA sequences U3A, U3B, U3C, and Katnal-2. Katnal 2 (katanin p60 subunit A-like 2) produces a protein that functions to promote rapid reorganization of cellular microtubules and was chosen as an irrelevant guide RNA control. Control PBMCs were not treated with any viral vector.

Three general patterns of cytokine/chemokine expression were observed. In one pattern, a particular cytokine/chemokine was not expressed in any of the infection/transduction conditions over the 72 hours. Cytokines that were not expressed included TGFα, FLT-3L, IL-12P70, PDGF-AA and PDGF-BB. The second pattern exemplified cytokines/chemokines that were constitutively secreted regardless of the stimulus (HIV-1, LV, or no treatment). These included EGF, FGF2, Eotaxin, GM-CSF, Fractaline, IL-10, IL-12P40, MDC, IL-13, IL-15, sCD40L, IL-17α, IL-1Rα, IL-3, IL-4, IL-5, IL-7, IP-10, MIP-1α, MIP-1β, RANTES, TNFα and TNF-β. IL-2 was present in all conditions, but because this cytokine was added to the PBMC media, no conclusion about induction of this cytokine can be assessed. The third pattern included cytokines that were expressed only after LV transduction in which the LV expressed the Cas9 gene with or without a guide RNA. As shown in Figure 6, those cytokines and chemokines that represented this third pattern of expression included G-CSF, IFN-α2, IFN-γ, GRO, MCP-3, IL-1α, IL-1β, IL-6, IL-8, IL-9, VEGF, and MCP-1. None of these 12 cytokines or chemokines were produced in any significant quantity in control conditions, or following exposure to HIV-1 or the FUGW LV.

### Example 11

### U3B Guide RNA Plus Cas9 Eliminates the Proviral Sequence in J-Lat Cells

J-Lat10.6 cells were used as a model for latent HIV-infected cells because they contain a modified integrated HIV-1 provirus that lacks several of the regulatory genes for HIV-1, but do include GFP and gag under the control of the LTR. Moreover, TNFα can activate proviral transcription in these J-Lat cells, leading to both GFP and p24 production. As e-LV and g-LV transduced clones of J-Lat also express GFP from the GFP Cas9 gene, we compared the dual fluorescence intensity profiles of J-Lat cells prior to and after TNFα activation. As shown in Figure 6A, parental (uncloned) J-Lat cells induce a bright peak of GFP in approximately 80% of the cells. Thus, parental cells do not express GFP in the native state, but can be induced to express high intensity GFP expression following TNF-α activation in approximately 80% of cells. Figure 6B shows both the less intense GFP peak from the integrated transgene from a representative J-Lat clone transduced with e-LV, and the induction of a brighter fluorescence peak from the integrated provirus following TNFα activation. Thus, a J-Lat clone transduced with e-LV expresses low intensity levels of GFP from the transgene, and a brighter intensity GFP following TNF-α activation. Figure 6C shows a representative J-Lat clone transduced with the g-LV, and lack of induction of the second, brighter GFP peak following TNFα activation. Thus, a J-Lat clone transduced with g-LV expresses low intensity levels of GFP from the transgene, and no induction of a brighter intensity GFP peak following TNF-α activation. Parent J-Lat and e-LV transduced clones, but not g-LV clones, also produced p24 secreted into the culture supernatant and levels of this protein is noted in the legend for Figures 6A-6C.

To determine the extent of proviral gene disruption in the g-LV-transduced clones compared to the e-LV clones, PCR assays were performed to amplify the region in the middle of the provirus, 4.5 kilobases from the U3 sequence targeted by the guide RNA. Electrophoretic analysis revealed an amplification product 176 bp in length when parental J-Lat cells provided the DNA template used for the PCR reaction. Likewise, the same band was observed when three J-Lat clones transduced with the e-LV construct (i*.e.*, did not encode HIV-1 specific gRNA) provided the DNA templates. Production of the 176 bp amplification product in each of these cases indicated the presence of HIV-1 proviral DNA. In contrast, the 176 bp amplification product was not synthesized when three J-Lat clones transduced with the g-LV construct (*i.e.*, encoded HIV-1 specific gRNA) provided the DNA templates. This result indicated that HIV-1 proviral sequences advantageously had been excised from the genome. A 239 bp amplification product of the CCR5 gene was detected in each of the seven trials, thereby validating the negative results in the trials evidencing excision of proviral DNA. Simply stated, the proviral sequence in the g-LV clones had been excised, but this region was present in its entirety in all e-LV clones analyzed.

The above description of the disclosed embodiments is provided to enable any person skilled in the art to make or use the invention. Various modifications to these embodiments will be readily apparent to those skilled in the art, and the generic principles described herein can be applied to other embodiments without departing from the spirit or scope of the invention. Thus, it is to be understood that the description and drawings presented herein represent a presently preferred embodiment of the invention and are therefore representative of the subject matter which is broadly contemplated by the present invention. It is further understood that the scope of the present invention fully encompasses other embodiments that may become obvious to those skilled in the art and that the scope of the present invention is accordingly not limited.

## Claims

1. A method of suppressing HIV-1 production in a peripheral blood mononuclear cell (PBMC) removed from a human subject, the method comprising the steps of:
contacting the PBMC with a composition comprising a viral vector, whereby the viral vector enters the PBMC,
wherein the viral vector comprises a nucleic acid sequence encoding a humanized Cas9 endonuclease and contains no proviral-specific gRNA sequence, and
wherein the PBMC is not contacted with a vector comprising a proviral-specific gRNA sequence.

2. The method of claim 1, wherein the viral vector is a lentiviral vector.

3. The method of claim 1, wherein the PBMC is selected from the group consisting of a lymphocyte and a macrophage, and wherein the composition is for use before or after the cell is infected with HIV-1 or the contacting step is performed before or after the cell is infected with HIV-1.

4. The method of claim 1, wherein the contacting step is performed before the cell is infected with HIV-1 or wherein the contacting step is performed after the cell is infected with HIV-1.

5. The method of claim 1, wherein the composition further comprises a pharmaceutically acceptable excipient.

6. The method of claim 2, wherein the lentiviral vector is packaged within a virion that comprises an envelope protein that binds to a specific cell type.


SEQUENCE LISTING

<110>  United States Government as Represented by the Department
       of Veterans Affairs

       Dartmouth College

<120>  LENTIVIRAL DELIVERY OF CRISPR/CAS CONSTRUCTS THAT CLEAVE GENES
       ESSENTIAL FOR HIV-1 INFECTION AND REPLICATION

<130>  JA97463P.EPPD1

<140>
<141>  2017-05-10

<150>  EP17796801.3
<151>  2017-05-10

<150>  PCT/US2017/032033
<151>  2017-05-10

<150>  62/334,313
<151>  2016-05-10

<160>  11

<170>  PatentIn version 3.5

<210>  1
<211>  82
<212>  DNA
<213>  Artificial Sequence

<220>
<223>  GUIDE RNA

<400>  1
guuuuagagc uagaaauagc aaguuaaaau aaggcuaguc cguuaucaac uugaaaaagu      60

ggcaccgagu cggugctttt tt                                               82


<210>  2
<211>  24
<212>  DNA
<213>  Artificial Sequence

<220>
<223>  FORWARD PRIMER

<400>  2
gataggtacc tggctgtcgt ccat                                             24


<210>  3
<211>  22
<212>  DNA
<213>  Artificial Sequence

<220>
<223>  REVERSE PRIMER

<400>  3

accagcccca agatgactat ct                                                22

<210>  4
<211>  20
<212>  DNA
<213>  Artificial Sequence

<220>
<223>  FORWARD PRIMER

<400>  4
ggcgttactc gacagaggag                                                    20

<210>  5
<211>  20
<212>  DNA
<213>  Artificial Sequence

<220>
<223>  REVERSE PRIMER

<400>  5
tcctgccata ggagatgcct                                                    20

<210>  6
<211>  23
<212>  DNA
<213>  Human immunodeficiency virus

<400>  6
tctacttgct ctggttcaac tgg                                                23

<210>  7
<211>  23
<212>  DNA
<213>  Human immunodeficiency virus

<400>  7
gggccatgtg acgaaatgct agg                                                23

<210>  8
<211>  23
<212>  DNA
<213>  Human immunodeficiency virus

<400>  8
cagcagtctt tgtagtactc cgg                                                23

<210>  9
<211>  20
<212>  DNA
<213>  Artificial Sequence

<220>
<223>  HIV IRRELEVANT

<400>  9

agcaggcaca ggtccgttgg                                                    20

<210>  10
<211>  102
<212>  DNA
<213>  Artificial Sequence

<220>
<223>  gRNA SCAFFOLD

<400>  10
ttttttcgug gcugagccac ggugaaaaag uucaacuauu gccugaucgg aauaaaauug          60

aacgauaaag aucgagauuu ugucguaaag caguguaccg gg                          102

<210>  11
<211>  23
<212>  DNA
<213>  Human immunodeficiency virus type 1

<400>  11
gggccatgtg acgaaatgct agg                                                23